# EUROPEAN PATENT SPECIFICATION

(11) **EP 3 707 147 B1**
(45) Date of publication and mention of the grant of the patent: **05.03.2025**
(21) Application number: 18808499.0
(22) Date of filing: 05.11.2018
(51) Int. Cl.: C07H 1/00, C07H 1/02, C07H 13/06, C07H 23/00, C07H 15/18, A61K 31/7024, A61K 31/7028, A61P 35/00, A61P 37/00, A61P 31/00

(54) **NEW SYNTHETIC AGONISTS OF TLR4 RECEPTOR**
NEUE SYNTHETISCHE AGONISTEN DES TLR4-REZEPTORS
NOUVEAUX AGONISTES SYNTHÉTIQUES DU RÉCEPTEUR TLR4

(30) Priority: 07.11.2017 IT 201700126612
(43) Date of publication of application: 16.09.2020
(73) Proprietor: Università degli Studi di Milano - Bicocca, 20126 Milano (IT)
(72) Inventor: PERI, Francesco, 20126 Milano (IT); MINOTTI, Alberto, 20126 Milano (IT)
(74) Representative: Predazzi, Valentina
(86) International application number: PCT/IB2018/058648
(87) International publication number: WO 2019/092572

(56) References cited:
- EP-A2- 0 309 411
- RIETSCHEL E T ET AL: "BACTERIAL ENDOTOXIN: MOLECULAR RELATIONSHIPS OF STRUCTURE TO ACTIVITY AND FUNCTION", THE FASEB JOURNAL, FEDERATION OF AMERICAN SOCIETIES FOR EXPERIMENTAL BIOLOGY, US, vol. 8, no. 2, 1 February 1994 (1994-02-01), pages 217 - 225, XP001148875, ISSN: 0892-6638

## Description

The present invention relates to new synthetic molecules with agonist activity of human Toll-like Receptor 4 (TLR4), compositions comprising them and uses thereof for the treatment of diseases in which it is useful to induce or increase an immune response.

### PRIOR ART

Receptors of immune system cells recognize a large variety of microbial pathogens, such as viruses, bacteria, fungi and parasites, via recognition of pathogen-associated molecular patterns (PAMPs) present on pathogens' surface.

Such receptors are known as pattern recognition receptors (PRRs) and may be of various typologies, depending on their localization in-cell, in cytosol or on the membrane, and on their function.

Toll-like receptors (TLRs) constitute the most well-known and studied family of innate immunity receptors, and have the role of promoting a rapid response to pathological menaces and aiding the development of adaptive immune response, which is the most appropriate and specific defense for such menaces.

In fact, innate immunity response to pathogens can be decisive in determining both the nature and the intensity of adaptive immunity response.

It is for that reason that TLR activators (agonists) have been studied and developed for the treatment of cancer, allergies and infective diseases, comprising adjuvants in prophylactic and therapeutic vaccines.

To date, there are various small molecules able to bind and activate TLR receptors, and some of those are in use as adjuvants: imidazoquinoline TLR7/8 agonists, like imiquimod and resiquimod, as well as Pam2CS-type TLR2/TLR6 agonist and TLR4 agonists such as monophosphoryl lipid A (MPL) and aminoalkyl glucosaminide-4-phosphates (AGPs, also referred to as Corixa compounds, CRX).

TLR4 receptor is a highly interesting target for the development of immunostimulants and vaccine adjuvants, as TLR4 stimulation by agonists is the most effective way to activate innate and adaptive immunities.

The natural agonist of TLR4 is lipopolysaccharide (LPS), the main component of the outer membrane of Gram-negative bacteria. Lipid A is the immunologically active portion of LPS.

Lipid A agonistic activity is based on its binding affinity (ability to bind) to the TLR4 co-receptor, Myeloid Differentiation factor 2, MD-2, with the entailed formation of the (TLR4/MD-2/LPS)₂ complex on the surface of innate immunity cells, i.e. macrophages and dendritic cells.

The activation process of the TLR4 receptor by LPS begins with the interaction of individual LPS molecules or aggregates in solution with Lipid Binding Protein (LBP), forming a complex with an LPS molecule. Thereafter, the LPS molecule is transferred from LBP to co-receptor CD14, which in turn transfers it from MD-2.

Natural endotoxins of bacteria, like LPS, lipooligosaccharides (LOS) and lipid A, are excessively toxic to be used as adjuvants.

Therefore, synthetic and natural proteins with a structure similar to lipid A, but with attenuated endotoxicity, are interesting candidates as vaccine adjuvants in the perspective of maintaining immunostimulatory activity while eliminating the toxic effects. Monophosphoryl lipid A (MPL) is a molecule identical to lipid A, but with the C₁ position stripped of the phosphate group through chemical modification. MPL has ~0.1% of the inflammatory toxicity of the parent molecule, LPS, and is used as adjuvant in a series of vaccines.

However, the MPL adjuvant used nowadays is chemically heterogeneous, as produced directly from natural LPS. The synthetic compounds named AGPs (also known as CRX adjuvants, Corixa) are comprised of a monosaccharide unit linked by glycosidation to a unit of an aminoalkyl aglycone N-acylate.

AGPs are potent agonists of TLR4 and are chemically homogeneous, as produced by chemical synthesis.

A further simplification of the structure of Lipid A still able to activate TLR4 is comprised of the monophosphorylated monosaccharide derivatives mimicking the reducing portion or the non-reducing portion of Lipid A (scheme below).

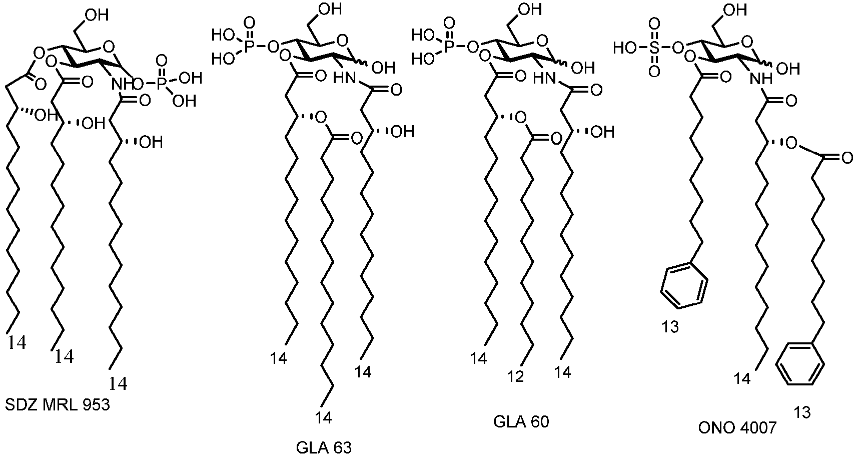

Compounds GLA 63 and GLA60 (scheme above) are comprised of a glucopyranoside skeleton, phosphorylated in position C₄ and with a 14-Carbon linear chain in C₂ and a branched chain in C₃ (14 + 14 in GLA 63 or 14 + 12 Carbons in GLA 60) (Motohiro Matsuura, Makoto Kiso and Akira Hasegawa Infect. Immun. 1999, 67(12), 6286-6292). These monosaccharides that partially mimic lipid A and mimic the monosaccharide lipid X, biosynthetic precursor of lipid A, are active in stimulating TLR4-dependent production of cytokines TNF-α and IL-6, in both murine and human cells.

Also compound SDZ MRL 953 demonstrated a potent activity in stimulating the release of inflammatory cytokines like interleukin-6 (IL-6), interleukin-8 (IL-8) and TNF-α factor in murine macrophages and neutrophil granulocytes, concomitantly exhibiting a toxicity reduced of a factor of at least 10⁴ in galactosamine-sensitized mice compared to the parent endotoxin *(Salmonella abortus equi*).

In experimental microbial infection models, the compound proved to have a highly protective effect when administered prophylactically either once or thrice in myelo-suppressed or immunocompetent mice.

Doses effective to reach 50% of response with SDZ MRL 953 vary depending on the infective agent and administration route. In all cases, however, EC₅₀ obtained are about 10³ times greater than those obtained with endotoxin *Salmonella abortus equi.*

However, thanks to the very low toxicity, the therapeutic indexes of this molecule, expressed, e.g., as LD₂₅/ED₇₅ were significantly improved compared to the endotoxin and range from about 5 to >500, depending on the infective agent and the administration route.

The compound also proved efficient in inducing tolerance to endotoxins: repeated dosages of the compound induce a transient resistance (≥1 week) to endotoxin-related lethal risks.

All these positive results were also confirmed in a model of advanced sepsis caused by *Escherichia coli,* in which antibiotic therapy had already proved inefficient: pre-treatment with one dose of SDZ MRL 953 one day prior to microbial inoculation dramatically increased the curative effects of antibiotics administered. For this reason, long-term survival was significantly increased with incremental doses of the immunostimulant in combined therapy.

Precisely for the tolerability demonstrated by SDZ MRL 953 in animals and *in vitro,* this compound was subsequently tested in human models.

On the basis of the known anti-tumour activity of *Salmonella abortus equi* endotoxin linked to its immunostimulating properties, Kiani et al. (A. Kiani, A. Tschiersch, E. Gaboriau, F. Otto, A. Seiz, H.-P. Knopf, P. Stütz, L. Färber, U. Haus, C. Galanos, R. Mertelsmann, and R. Engelhardt, Blood, 1997, 1673-1683) conducted a randomized double-blind phase I trial with control medium, administering SDZ MRL 953 in tumour-affected patients in order to assess firstly its biological effects and its safety of administration in humans and, secondly, its influence on the reaction to a subsequent endotoxin (LPS) addition.

SDZ MRL 953 administration proved safe and of excellent tolerability. The same SDZ MRL 953 increases granulocyte counts and serum levels of G-CSF and interleukin-6 (IL-6), but not of pro-inflammatory cytokines TNF-α, IL-1b, and IL-8.

Therefore, SDZ MRL 953 has three relevant features, i.e., 1) a high tolerability and low toxicity, 2) the ability to induce G-CSF production, and, as a result, 3) the ability to stimulate an aspecific immune resistance expressed by an increased group of primary defenses in cells.

In spite of these positive results encouraging in the clinical use of SDZ MRL 953, the action mechanism of this molecule has not yet been studied in molecular detail.

*In vitro* studies suggest that the compound acts independently of co-receptor CD14, as by using monoclonal antibodies for CD-14 the compound-induced TNF-α release in human peripheral blood cells is not blocked.

This in turn seems to be indicative of a direct interaction with compound MD-2/TLR4, and might explain its different cytokine induction profile.

The synthesis of compound SDZ MRL 953 is made complex by the fact that the glucosamine core binds, in positions C₂, C₃ and C₄, chains of 3(R)-hydroxymyristic acid as pure enantiomer. 3-hydroxymyristic acid is in fact commercially available as racemate, whereas the pure enantiomer 3(R)- hydroxymyristic acid needs to be first isolated, to then be used in the synthesis.

EP 0309411 A2 discloses compounds having formula I 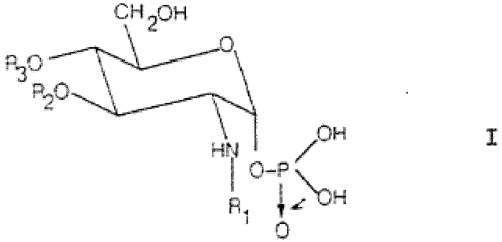 wherein R1, R2 and R3 independently are optionally substituted acyl, and describes a process for the preparation of the compound of formula I, and its use as pharmaceuticals, in particular for use as immunomodulators, as antiviral agents and as anti-inflammatory agents, and for use in the treatment of allergies.

### SUMMARY OF THE INVENTION

The Authors of the present invention have surprisingly found that compounds of formula 1 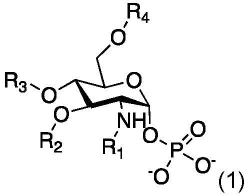
wherein R₁ is a saturated C₈-C₁₆ aliphatic chain having a =O on C₁, said chain being free from -OH substituents on C₃,
wherein R₂ is a saturated C₈-C₁₆ aliphatic chain having a =O on C₁, said chain being free from -OH substituents on C₃,
wherein R₃ is a saturated C₈-C₁₆ aliphatic chain having a =O on C₁, said chain being free from -OH substituents on C₃,
wherein R₄ is a hydrogen atom (H) or O-R₄ is a phosphate group (PO₄²⁻), and
wherein R1, R2 or R3 have no other substituents besides =O in C1 position;

said compounds are particularly effective in activating the TLR4 receptor and require synthesis processes that are less complex compared to compounds of the known art. In fact, the present invention enabled to identify compounds requiring simpler synthetic pathways, that are equally effective, or even more effective compared to those present in the known art.

Therefore, object of the invention are compounds of formula 1 as TLR4 receptor agonists, and their use as active principle or as adjuvant in the treatment of diseases requiring a TLR4 receptor activation, as well as drug or vaccine compositions comprising said compound.

### DETAILED DESCRIPTION OF THE FIGURES

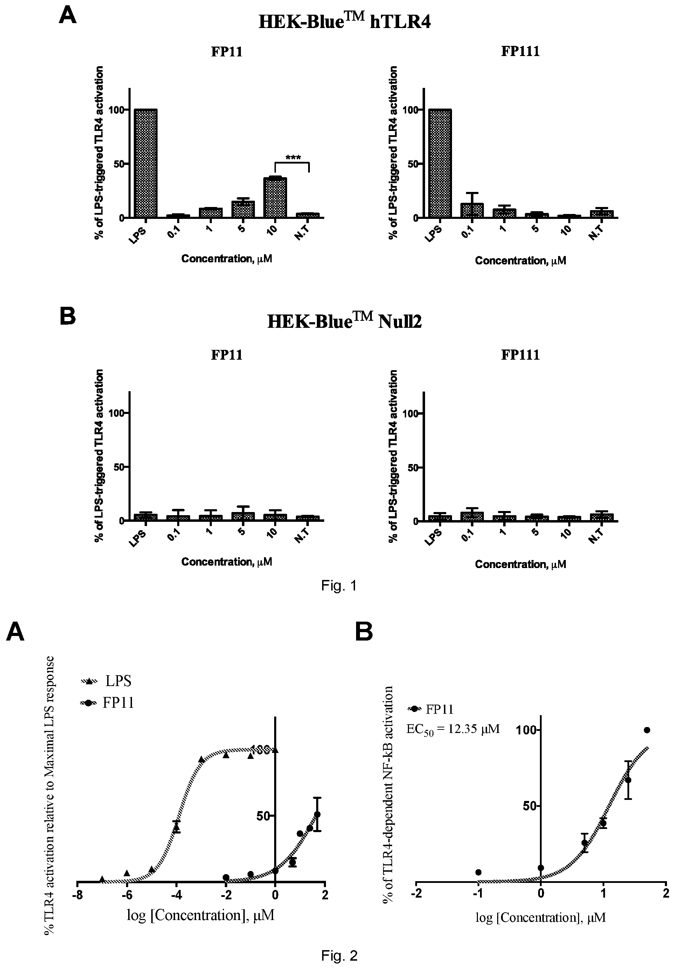

### Figure 1. Effect of compounds FP11 and FP111 on HEK-Blue^{™} hTLR4 and HEK-Blue^{™} Null2 cells.

HEK-Blue^{™} hTLR4 **(A)** and HEK-Blue^{™} Null2 **(B)** cells were treated with the indicated concentrations of compounds FP11, FP111, and with LPS (100 ng/mL) and incubated for 16-18 hours. Results were normalized to stimulation with LPS alone, and are expressed as mean of percentage ± ES. of at least three independent experiments.

### Figure 2. Dose-response curve of compound FP11 obtained on HEK-Blue^{™} hTLR4 cells.

HEK-Blue^{™} hTLR4 cells were treated with increasing concentrations of compound FP11 and of LPS and incubated for 16-18 hours. Results were normalized to maximum activation of the reporter produced by LPS **(A)** and to maximum activation of the reporter produced by the compound itself **(B).** Data were fitted to a sigmoidal four-parameter logistic equation in order to determine IC₅₀ values, and expressed as mean of percentage ± ES of at least three independent experiments.

### Figure 3. Effect of Compound FP11 on HEK-Blue^{™} hTLR4 cell viability.

HEK-Blue^{™} hTLR4 cells were treated with the higher concentrations used in the preceding assays and incubated for 16-18 hours. Results were normalized to PBS addition and are expressed as mean of percentage ± ES. of at least three independent experiments.

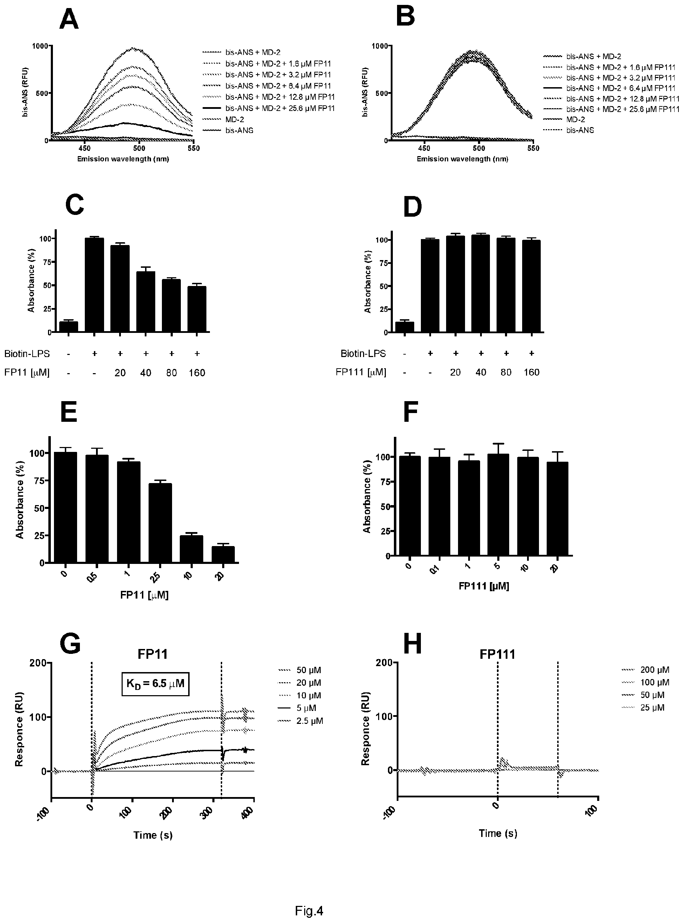

### Figure 4. Binding studies of synthetic compounds FP11 and FP111 with purified human MD-2 receptor

**(A-B)** Fluorescent measurements demonstrate that FP11, but not FP111, inhibits the binding of bis-ANS to MD-2 in a dose-dependent manner; **(C-D)** FP11, but not FP111, displaces biotinylated LPS from MD-2 hydrophobic pocket in a dose-dependent manner; **(E-F)** FP11, but not FP111, prevents the binding of monoclonal antibody to MD-2 in a dose-dependent manner; (G-H) SPR experiments indicate that FP11, but not FP111, directly binds MD-2 with a K_{D} value of 6.5 µM

### GLOSSARY

To the ends of the present description, the term "TLR4 receptor agonist" denotes a compound that selectively binds to the TLR4 receptor inducing a conformational change of said receptor, in turn generating an intracellular stimulation by triggering a response similar to that induced by the natural ligand of said receptor. In the case of TLR4, the substances described as agonists bind to co-receptor MD-2, in turn non-covalently bound to TLR4, thereby generating the receptorial complex (TLR4/MD-2/agonist)₂, which from the cell surface initiates a signal cascade leading to activation of nuclear transcription factors and synthesis of pro-inflammatory cytokines (mainly TNF-α and various interleukin types).

### DETAILED DESCRIPTION OF THE INVENTION

The present invention relates to a compound of formula 1 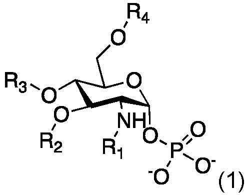
wherein R₁ is a saturated C₈-C₁₆ aliphatic chain having a =O on C₁, said chain being free from -OH substituents on C₃,
wherein R₂ is a saturated C₈-C₁₆ aliphatic chain having a =O on C₁, said chain being free from -OH substituents on C₃,
wherein R₃ is a saturated C₈-C₁₆ aliphatic chain having a =O on C₁, said chain being free from -OH substituents on C₃.
wherein R₄ is a hydrogen atom (H) or O-R₄ is a phosphate group (PO₄²⁻), and
wherein R1, R2 or R3 have no other substituents besides =O in C1 position;

Therefore, according to the present description, each aliphatic chain, R₁, R₂, or R₃, can have a length of C₈-C₁₆.

According to the description, R₁, R₂, and R₃ can be different from each other or alike to each other.

In one embodiment of the invention, R₁, R₂, and R₃ are alike to each other.

According to some possible non-limiting embodiments, the compound of formula 1 could be selected from the following ones:
Compound FP11: with R₁ = R₂ = R₃ = C=OC₁₃H₂₇ R₄ = H
compound FP111: with R₁ = R₂ = R₃ = C=OC₁₃H₂₇ R₄ = PO₄²⁻
compound FP112: with R₁ = R₂ = R₃ = C=OC₁₁H₂₃ R₄ = H
compound FP113: with R₁ = R₂ = R₃ = C=OC₁₁H₂₃ R₄ = PO₄²⁻
compound FP114: with R₁ = R₂ = R₃ = C=OC₉H₁₉ R₄ = H
compound FP115: with R₁ = R₂ = R₃ = C=OC₉H₁₉ R₄ = PO₄²⁻

In one preferred embodiment, the compound of the invention is the compound having formula

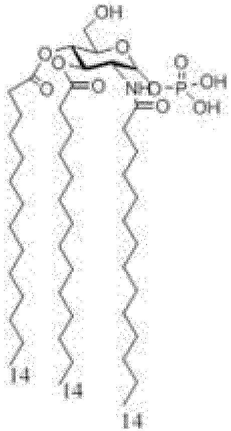

In the present description, such a compound is also referred to as compound FP11.

Data reported in the Examples section show the peculiar advantageous features of the above-indicated compounds FP.

According to the present description, and on the basis of experimental data obtained, it is evident that the compounds as described and claimed are effective agonists of TLR4 receptor. By "agonist of a receptor" (receptor agonist) it is meant as is commonly defined in the literature, i.e., a substance able to bind a specific receptor in the binding site for the endogenous ligand. Therefore, as the name suggests, the former competes with the latter for the binding with said site.

Following binding with the natural ligand, the receptor encounters conformational changes that mediate its biological activity at cell level. Agonists are molecules having inherent activity able to mimic ligand effects. When binding to the receptor, they cause conformational changes of an extent similar to those caused by binding with the endogenous ligand.

In the case of the present description, the agonist is an agonist selective for the TLR4 receptor.

Preliminary data (not shown) obtained by the Authors of the invention show that the compounds of formula 1 are more effective than analogous compounds having-OH groups bound on C₃ known in the literature, like, e.g., the compound described in the state of the art, similar to the compound FP11 but having -OH groups on the C₃ of each R chain.

Given the technical features observed for compounds of formula (1) as defined in the present description and in the claims, said compounds are useful as active principles or as adjuvants in diseases benefiting from a TLR4 receptor activation, i.e., in diseases in which an activation of the immune system, particularly of the innate activity, is therapeutic or prophylactic.

Therefore, by diseases requiring or benefiting from a TLR4 receptor activation, diseases are meant whose treatment or whose prevention are improved by a TLR4 receptor activation and by the related innate immune response triggered by the activation of said receptor. A non-limiting example of such diseases is represented by tumours.

An additional particularly advantageous use of the compound of formula 1 in any one of the embodiments provided in the description or in the claims is that as vaccine adjuvant. In fact, the relevance of immune response adjuvants during vaccine administration is known. Such adjuvants, in fact, substantially increase vaccine effectiveness and development of immunity, in the treated subject, toward antigens present in the vaccine.

Therefore, object of the present invention is also a vaccine composition comprising the compound of formula 1 in any one of the embodiments provided in the description or in the claims.

The vaccine composition according to the invention could therefore comprise the compound of formula 1 as described herein, in any one of the above-listed embodiments, at least one pharmaceutically acceptable carrier and at least one antigenic compound able to induce an immune response to a given pathology.

The composition could be prepared for a single administration of adjuvant and antigen, or for a concomitant administration thereof.

According to another embodiment, the present invention relates to a pharmaceutical composition for use in the treatment of diseases that require or benefit from a TLR4 receptor activation, comprising the compound of formula 1 in any one of the embodiments provided in the description or in the claims and at least one pharmaceutically acceptable excipient.

Said pharmaceutical composition can also be formulated as association between plural active principles.

According to the present description, said diseases may be cancer, allergies or infective diseases.

The pharmaceutical composition of the invention could comprise as sole active principle one or more compounds of formula 1 in any one of the embodiments provided in the description or in the claims, or could also comprise further active principles, such as anti-tumour active principles, kinase inhibitors, cytotoxic compounds and at least one pharmaceutically acceptable carrier or excipient.

The composition could be for oral or injectable use, and suitable conventional carriers and/or excipients for liquid, semiliquid, solid formulations, granules or others can be selected by the technician in the field.

According to the invention, the composition could comprise 0.5 to 10 mg of compound of the invention per daily dosage: 1 to 10 mg of substance per Kg (test on animals).

The compounds of formula 1, in particular FP11, can be synthesized in a simpler and industrially scalable way compared to compound SDZ MRL953. The latter requires the insertion of three acyl chains of (R)-3-hydroxymyristic acid. The optically pure compound (R-enantiomer) is not commercially available, as only the racemic mixture is marketed. Moreover, (R)-3-hydroxymyristic acid requires a reaction of protection of the hydroxyl group in 3 position prior to the condensation reaction with the sugar. Compound FP11 synthesis is therefore remarkably simplified by the use of non-hydroxylated myristic acid chains.

The compounds provided in the present invention exhibit activities comparable to, if not even better than the above-reported compound SDZ MRL953, despite differences, and can be synthesized in a much simpler and industrially scalable way. Therefore, the present invention also relates to a method of synthesis for the preparation of compounds of formula 1 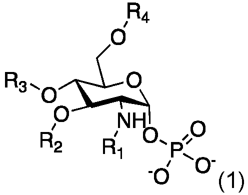
wherein R₁ is a saturated C₈-C₁₆ aliphatic chain having a =O on C₁, said chain being free from -OH substituents on C₃,
wherein R₂ is a saturated C₈-C₁₆ aliphatic chain having a =O on C₁, said chain being free from -OH substituents on C₃,
wherein R₃ is a saturated C₈-C₁₆ aliphatic chain having a =O on C₁, said chain being free from -OH substituents on C₃.
wherein R₄ is a hydrogen atom (H) or O-R₄ is a phosphate group (PO₄²⁻), and
wherein R1, R2 or R3 have no other substituents besides =O in C1 position;
   wherein each aliphatic chain, R₁, R₂, or R₃, can have a length of C₈-C₁₆.

According to some possible non-limiting embodiments, the compound of formula 1 could be selected from the following:
compound FP 11: with R₁ = R₂ = R₃ = C=OC₁₃H₂₇ R₄ = H
compound FP111: with R₁ = R₂ = R₃ = C=OC₁₃H₂₇ R₄ = PO₄²⁻
compound FP112: with R₁ = R₂ = R₃ = C=OC₁₁H₂₃ R₄ = H
compound FP113: with R₁ = R₂ = R₃ = C=OC₁₁H₂₃ R₄ = PO₄²⁻
compound FP114: with R₁ = R₂ = R₃ = C=OC₉H₁₉ R₄ = H
compound FP115: with R₁ = R₂ = R₃ = C=OC₉H₁₉ R₄ = PO₄²⁻

In one preferred embodiment, the compound of the invention is the compound having formula

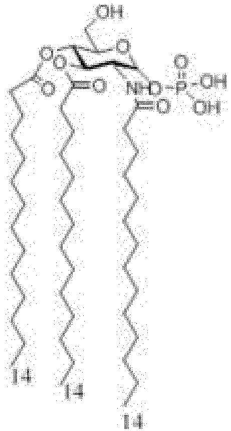

Also referred to herein as FP11.

The synthetic method enabling to obtain the compounds having formula 1, like, e.g., FP11 and the variants indicated herein as FP111-115, comprises the following steps (also reported in Scheme 1):
1) transformation of the amine group of glucosamine into azide by reaction with trifluoromethansulfonic (triflic) azide;
2) selective protection of C₄ and C₆ hydroxyls on the sugar of said glucosamine forming para-methoxybenzylidene cyclic acetal by reaction with para-methoxybenzaldehyde dimethylacetal in the presence of camphorsulfonic acid (CSA); the forming of said benzylidene can also be carried out from para-methoxybenzaldehyde in the presence of a catalyst acid;
3) protection of the anomeric carbon (C₁) as tert-butyldisilyl (TBS) ether, e.g., by reaction with TBS chloride in the presence of imidazole;
4) acylation of C₂ and C₃ positions with a C₈-C₁₆ linear chain carboxylic acid in the presence of condensing agents, such as, e.g., 1-Ethyl-3-(3-dimethylaminopropyl) carbodiimide (EDC) in the presence of N,N'-dimethylamino pyridine (DMAP);
5) regioselective opening of the 4,6-paramethoxybenzylidene in reducing conditions to give para-methoxybenzyl ether in C₆ position via reaction with sodium cyanoborohydride and hydrochloric acid;
6) acylation of the C₄ position by reaction with a C₈-C₁₆ linear chain carboxylic acid in the presence of condensing agents, e.g. 1-Ethyl-3-(3-dimethylaminopropyl) carbodiimide (EDC) in the presence of dimethylaminopyridine (DMAP);
7) desylilation of the (anomeric) hydroxyl on C₁ by reaction with tetrabutylammonium fluoride (TBAF);
8) phosphorylation of position C₁ by reaction with dibenzyl-diisopropylphosphoramidite and imidazolium triflate, followed by phosphorous oxidation by the use of *meta-*chloroperbenzoic acid;
9) deblocking the para-methoxybenzyl ether in C₆ and simultaneously deprotecting the benzyl groups on the phosphates by hydrogenation in the presence of palladium-carbon catalyst.

The above-described method enables the synthesis/preparation of monophosphorylated derivatives of the compounds of formula 1, like FP11, FP112 and FP114.

Alternatively, to have the compounds with two phosphate groups in positions C₁ and C₆, after phosphorylation of the position described in 8), the following steps are carried out: 10) deblocking the para-methoxybenzyl ether in C₆ by catalytic hydrogenation with a Pd/C catalyst
11) phosphorylation in C₆ by reaction with dibenzyl-*N,N*-diisopropylphosphoramidite and imidazolium triflate, followed by phosphorous oxidation by the use of *meta-*chloroperbenzoic acid; and,
12) deprotection of benzyl groups on the phosphates by catalytic hydrogenation with a Pd/C catalyst.

According to the present description, the protection of hydroxyls at 2) can be carried out according to techniques commonly used by the technician in the field, like, e.g., reaction with paramethoxy acetaldheyde or dimethyl acetal thereof in the presence of a catalyst acid like camphorsulfonic acid (CSA).

The protection of the anomeric carbon at 3) can be carried out by any suitable technique known to a technique in the field, like, e.g., sylilation of the hydroxyl on C₁ by reaction with tetrabutylammonium fluoride (TBAF).

Acylation by condensation with various linear-chain carboxylic acids at 4) and/or 6) enables the synthesis of variants with Carbon chains of different length on C₂ and C₃ positions, depending on the acids used.

The condensing agents at 4) or 6) can be any suitable condensing agent known to the technician in the field, like, e.g., dicyclohexylcarbodiimide (DCC) or 1-Ethyl-3-(3-dimethylaminopropyl) carbodiimide (EDC).

According to the present description, it is possible to use C₁₀-C₁₆ linear chain carboxylic acids; in one embodiment, leading to the synthesis of compound FP11, myristic acid is used in both steps.

The regioselective opening in reducing conditions according to 5) can be carried out according to any suitable technique commonly used by the technician in the field, like, e.g., sodium cyanoborohydride and hydrochloric acid.

The process of synthesis according to the invention enables to make in an easy and industrially scalable way the compounds of formula 1.

Object of the invention is also a process for the preparation of pharmaceutical formulations or of vaccine compositions comprising the steps of the above process, and at least one step wherein the product obtained at 9) or 12) in a pharmaceutically acceptable form is mixed with at least one pharmaceutically acceptable adjuvant or excipient.

### EXAMPLES

### Example 1. Synthesis of Compound FP11 and of Compound FP111

Compound FP11, agonist of TLR4 (scheme below) having three myristic acid chains (C₁₄) linked at positions C₂, C₃ and C₄ of the sugar and a phosphate group in C₁ in α-anomeric configuration.

### This compound is free from hydroxyl groups on C₃ of the lipophilic chains

The compound FP111, having a second phosphate group in C₆, was also synthesized.

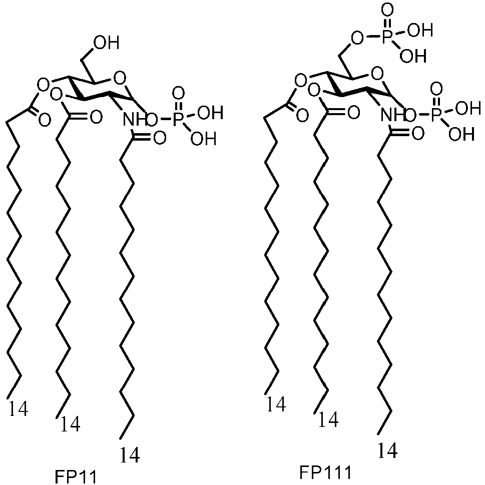

Compounds FP11 and FP111 were synthesized according to the reaction scheme reported below.

Commercially available D-glucosamine was first of all transformed, through 5 steps, into intermediate 5, which has two myristic acid chains in C₂ and C₃, and is protected as p-methoxybenzylidene in positions C₄ and C₆.

Regioselective opening of the benzyldene ring with sodium cyanoborohydride and HCl enables to obtain the compound 6 with a free hydroxyl in position 4.

The third esterification in this position, followed by C₁ deprotection with TBAF provides intermediate 8.

From this common intermediate, phosphorylation produces intermediate 9, which in turn, subjected to hydrogenation, is deblocked of all benzyl groups, on the phosphate, and paramethoxybenzyl groups, on C₆, to yield compound FP11; otherwise, hydrogenation before phosphorylation frees also position 6, enabling the insertion of two phosphate groups, whose deprotection yields compound FP111.

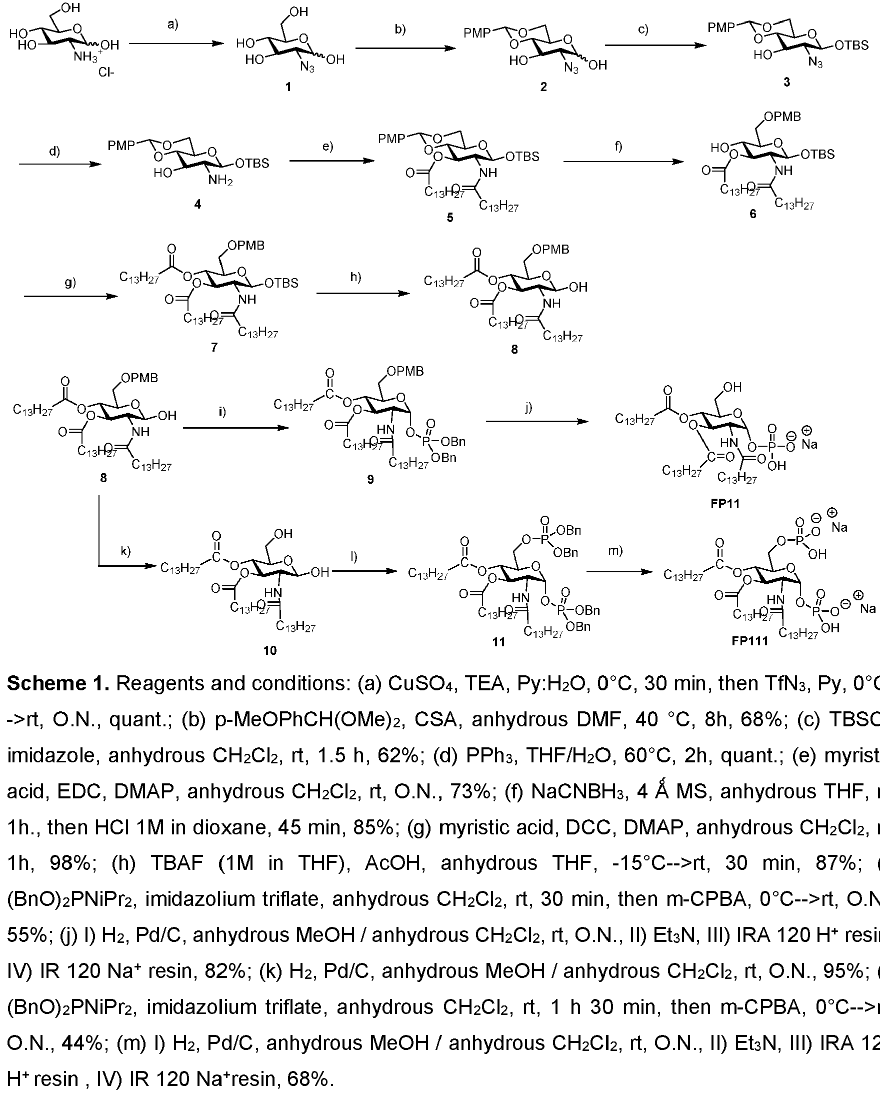

### Example 2.

### FP11 and FP111 monosaccharides were tested for their ability to activate TLR4 in HEK-Blue^{™} hTLR4 cells.

HEK-BlueTM hTLR4 cells are HEK cells transfected so as to stably express human receptors hTLR4, hMD-2, and hCD14 for endotoxin recognition. In addition, said cells have a reporter gene encoding for a secreted alkaline phosphatase (SEAP) placed under the control of specific transcription factors (NF-κB and AP-1) activated by the TLR4 signaling pathway. Therefore, the presence of LPS or of an agonist of TLR4 causes receptor dimerization, activation of the signaling pathway and of transcription factors NF-κB and AP-1, and finally SEAP production and secretion into the culture medium. SEAP levels can be subsequently quantitated by incubating the medium with compound p-nitrophenylphosphate (pNPP) and monitoring with a spectrophotometer the formation of p-nitrophenol chromogenic product at 405 nm.

The test conducted on HEK-BlueTM hTLR4 cells shows that compound FP11 is able to induce activation of NF-κB e AP-1 at concentrations of >5 µM. Conversely, compound FP111 proved completely inactive, demonstrating how the number of phosphate groups plays a crucial role in determining the agonist activity of the compound (Figure 1A). In order to be certain that the agonist effect of compound FP11 be due to the interaction with the TLR4 receptor, the compounds were tested on the HEK-Blue^{™} Null2 cell line. Said line expresses the same reporter gene of HEK-BlueTM hTLR4 cells (SEAP), but has none of the receptors involved in endotoxin recognition (TLR4, MD-2, CD14). Both compounds tested proved unable to activate transcription factors NF-κB and AP-1, demonstrating how FP11 effect is due to interaction with the LPS receptor complex (Figure 1B).

### Example 3

### Assessment of extent of TLR4 agonist activity of Compound FP11

To determine the extent of the agonist activity of compound FP11, two dose-response curves were constructed (Figure 2): the first curve shows FP11 activity in connection with LPS (Figure 2A), whereas the second curve indicates the compound activity normalized on its maximum TLR4 activation power (Figure 2B). EC₅₀ value calculated for FP11 (12.35 µM) is remarkably greater than that of LPS (0.13 nM), causing a more moderate activation of TLR4 signaling pathway.

### Example 4

### Assessment of Compound FP11 cytotoxicity

Finally, compound FP11 cytotoxicity was assessed by MTT (3-(4,5-dimethylthiazol-2-yl)-2,5-diphenyltetrazolium bromide) viability assay. HEK-BlueTM hTLR4 cells were treated with the highest compound concentrations used in the preceding assay (25 and 50 µM). The MTT assay reveals that compound FP11 is not toxic at the concentrations assayed.

### Example 5

### Binding studies of synthetic Compounds FP11 and FP111 with purified human MD-2 receptor

Interaction studies of synthetic molecules FP11 and FP111 with purified human MD-2 receptor were carried out by using four different techniques: two ELISA method-based assays with blocked MD-2, a fluorescent molecule displacement assay and SPR measurements.

### MD-2 - bound fluorescent molecule displacement assay

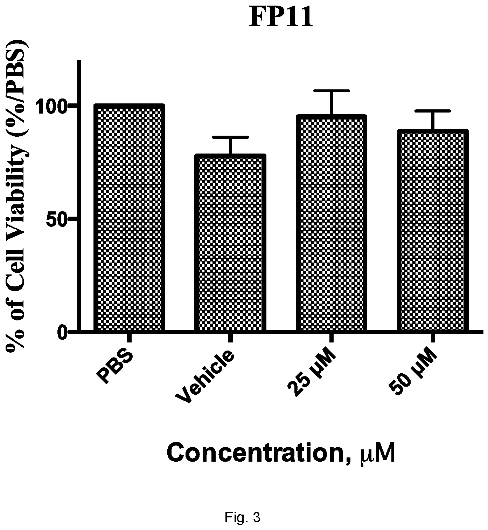

It has been demonstrated that compound 1,1'-Bis(anilino)-4,4'-bis (naphtalene)-8,8' disulfonate (bis-ANS) binds MD-2 and is displaced by LPS (Mancek-Keber, M. and R. Jerala, Structural similarity between the hydrophobic fluorescent probe and lipid A as a ligand of MD-2. Faseb j, 2006. 20(11): p. 1836-42). bis-ANS presumably binds the same hydrophobic pocket of MD-2 accountable for the binding of lipid A lipophilic chains; therefore, TLR4 modulators that interact with MD-2 compete with compound bis-ANS, and are able to displace it from MD-2. FP11 causes a concentration-dependent decrease of compound bis-ANS fluorescence, indicating a competitive-type binding of FP11 to MD-2 (Figure 3A). FP111 is not able to induce a decrease of compound bis-ANS fluorescence at the assayed concentrations (Figure 3B).

### Experiments of biotinylated LPS displacement from MD-2 hydrophobic pocket

The ability to displace LPS from MD-2 hydrophobic pocket was assessed by using an ELISA assay. Molecules FP11 and FP111 were added at increasing concentrations to MD-2 which had previously been incubated with biotinylated LPS. FP11 exhibits the ability to displace biotinylated LPS from MD-2 hydrophobic pocket in a dose-dependent manner, with a 55% displacement obtained at a concentration of 160 µM (Figure 3C). FP111 was unable to displace biotinylated LPS at the highest concentration assayed, of 160 µM (Figure 3D).

### Anti-MD-2 antibody competition experiments

The direct binding of molecules FP11 and FP111 to MD-2 was analyzed by using a monoclonal antibody binding MD-2, but not LPS-bound MD-2 (Viriyakosol, S., et al., Characterization of monoclonal antibodies to human soluble MD-2 protein. Hybridoma (Larchmt), 2006. 25(6): p. 349-57).

Anti-MD-2 monoclonal antibody (9B4) specifically binds an epitope near the MD-2 hydrophobic pocket, which is available for 9B4 antibody binding only when the MD-2 hydrophobic pocket is empty. Compound FP11 is accountable for a decrease of 9B4 antibody binding to MD-2 equal to 95% at a concentration of 20 µM (Figure 3E). FP111 was unable to cause a decrease of 9B4 antibody binding at the concentration of 20 µM (Figure 3F).

### Characterization of binding between MD-2 and the molecules by using surface plasmon resonance

Surface plasmon resonance (SPR) experiments enable the study of direct interactions between the molecules to be characterized and MD-2. SPR data show binding interactions between FP11 and MD-2, and the results indicate that FP11, but not FP111, directly binds MD-2 with a K_{D} value of 6.5 µM (Figure 3G-H).

These results obtained from the 4 *in vitro* assays on purified MD-2 receptor clearly demonstrate that FP11 directly binds the MD-2 hydrophobic pocket, whereas the molecule FP111 does not bind MD-2.

### POSSIBLE SYNTHESIS PATHWAYS OF THE OTHER COMPOUNDS INDICATED IN

THE CLAIMS: FP112, FP113, FP114, FP115

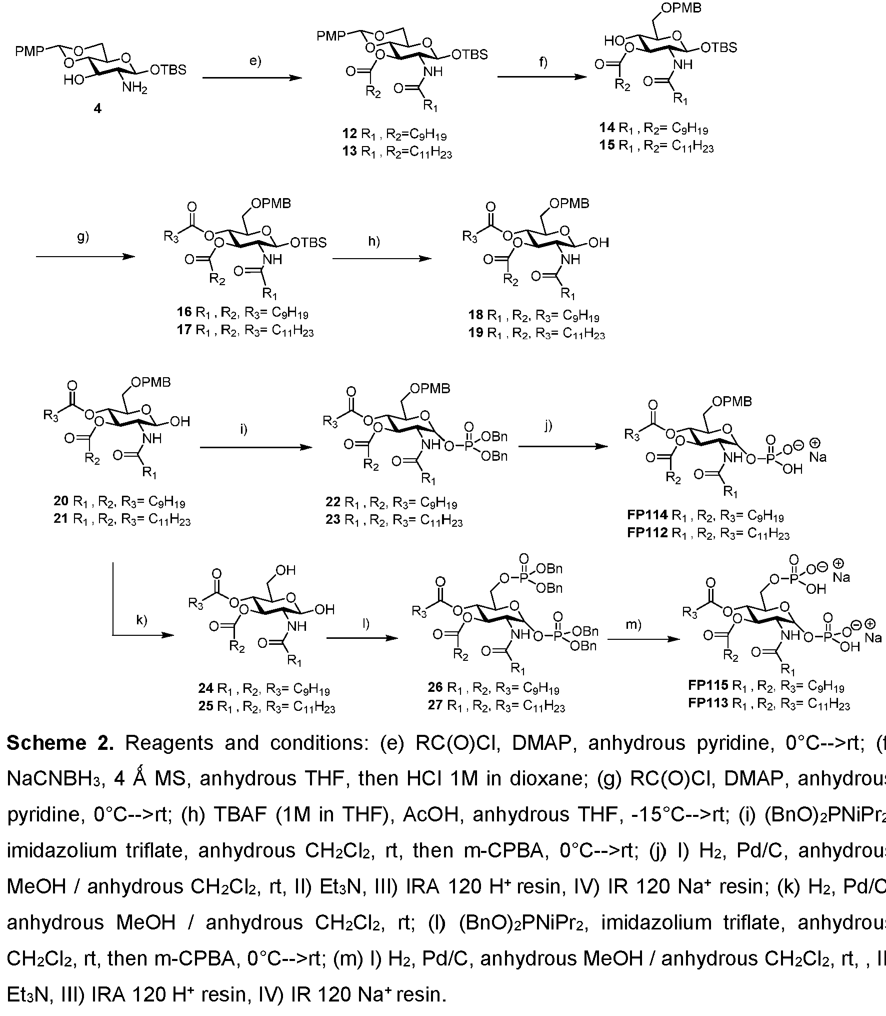

### Materials and methods

### CHEMISTRY

All reagents were available on the market and used without further purifications, unless noted otherwise.

When dry solvents were used, the reactions were carried out in stove-dried glassware under light argon pressure.

The reactions were magnetically stirred.

The reactions were monitored by thin-layer chromatography (TLC) on silica gel. TLC was performed on 60 F254 plates of silica gel (Merck). Spot revealing was carried out using UV light (254 nm) or a molibdate development solution [aqueous H₂SO₄ (5%) with (NH₄)₆Mo₄O₇·4H₂O (4%) and 0.2% Ce(SO₄)₂] or a H₂SO₄ solution [H₂O (45%) 6 EtOH (45%) with aqueous H₂SO₄ (10%)], followed by heating to 120°C.

A flash column chromatography was performed on 230-400 mesh (Merck) silica gel. The petroleum ether used as eluent in the chromatography has a boiling range of 40-60°C.

¹H and ¹³C NMR spectra were recorded with a Varian 400 MHz Mercury instrument at 298 K. Chemical shifts are reported in ppm downfield from TMS as internal standard. Mass spectra were recorded with an ESI-MS triple quadrupole instrument (API2000 QTrap model, Applied Biosystems).

### 1-O-tert-butyldimethylsylil-2-deoxy-6-O-(4-methoxybenzyl)- 4-O-tetradecanoyl 3-O-tetradecanoyl-2-tetradecanoylamino- β -D-glucopiranoside (7).

Myristic acid (144 mg, 0.63 mmol) was dissolved in anhydrous CH₂Cl₂ (3 mL) and DCC (195 mg, 0.95 mmol) was added. After 30 min, compound **6** (scheme 1 above) (263 mg, 0.32 mmol) dissolved in anhydrous CH₂Cl₂ (2 mL) and DMAP (39 mg, 0.32 mmol) were added and the mixture was mixed at room temperature for 1 hour. Precipitate was removed by filtration and solvents evaporated under vacuum. The raw product was purified by flash chromatography (petroleum ether-AE 9:1) to yield compound 7 (scheme 1 above) as a white solid (323 mg, 98%).

¹H-NMR: (400 MHz, CDCl₃, 25 °C, TMS): δ= 7.23 (d, ³J(H,H) = 8.4 Hz, 1H, 2x H-ortho), 6.85 (d, 3J(H,H) = 8.4 Hz, 1H, 2x H-meta), 5.30 (d, 1H, NH), 5.15 (t, ³J(H,H) = 10.2 Hz, 1H, H-3), 5.03 (t, 3J(H,H) = 9.6 Hz, 1H, H-4), 4.77 (d, ³J(H,H) = 8.1 Hz, 1H, H-1), 4.45 (s, 2H, CH₂-PMP), 3.96 - 3.85 (m, 1H, H-2), 3.79 (s, 3H, OCH₃), 3.66 - 3.58 (m, 1H, H-5), 3.51 (m, 2H, H-6a, H-6b), 2.23 (t, ³J(H,H) = 7.8 Hz, 2H, CH₂α-chain1), 2.14 (t, ³J(H,H) = 7.6 Hz, 2H, CH₂α-chain2), 2.10 - 2.02 (m, 2H, CH₂α-chain3),1.63 - 1.43 (m, 6H, CH₂β-chains1,2,3), 1.24 (m, 60H, 30xCH2), 0.94 - 0.79 (m, 15H, 2xCH₃- chains1,2 ,t-Bu-Si), 0.12 (s, 3H; CH₃-Si), 0.08 (s, 3H; CH₃-Si).

¹³C NMR (101 MHz, CDCl₃, 25 °C, TMS) δ 173.90, 172.60, 172.26, 159.14, 129.98, 129.27, 129.25, 113.67, 96.38, 73.50, 73.19, 72.31, 69.32, 56.27, 55.23, 55.22, 36.91, 34.18, 34.11, 31.92, 29.67, 29.65, 29.49, 29.47, 29.36, 29.31, 29.29, 29.17, 29.13, 26.40, 25.58, 24.94, 24.79, 22.70, 22.68, 17.88, 14.13, -4.02, -5.23.

### 2-deoxy-6-O-(4-methoxybenzyl)- 4-O-tetradecanoyl 3-O-tetradecanoyl-2-tetradecanoylamino- β -D-glucopiranoside (compound 8, scheme 1 above).

Compound 7, scheme 1 above (323 mg, 0.31 mmol) was dissolved in anhydrous THF (15 mL), cooled to -15 °C, and a solution of TBAF (107 mg, 0.34 mmol) and AcOH (22 µl, 0.39 mmol) in THF (340 µL) was added. The reaction was mixed at -15° C for 10 min, then left warming to room temperature and left mixing at room temperature for 30 min. The solution was diluted in water and extracted with CH₂Cl₂. Organic layer was dried with Na₂SO₄, filtered, and solvents were evaporated under vacuum. The raw product was purified by flash chromatography (petroleum ether-AE 7:3) to yield compound 8 as a red oil (250 mg, 87%).
¹H-NMR: (400 MHz, CDCl₃, 25 °C, TMS): 7.24 (d, ³J(H,H) = 8.7 Hz, 1H, 2x H-ortho), 6.85 (d, ³J(H,H) = 8.6 Hz, 1H, 2x H-meta), 5.71 (d, ³J(H,H) = 9.5 Hz, 1H, H-1), 5.34 - 5.23 (m, 2H, NH, H-3), 5.07 (t, ³J(H,H) = 9.9 Hz, 1H, H-4), 4.45 (s, 2H, CH₂-PMP), 4.32 - 4.24 (m, 1H, H-2), 4.19 - 4.11 (m, 1H, H-5), 3.79 (s, 3H, OCH₃), 3.55 - 3.38 (m, 1H, H-6a, H-6b), 3.02 - 2.84 (bs, 1H, OH), 2.22 (t, ³J(H,H) = 7.6 Hz, 2H, CH₂α-chain1), 2.18 - 2.05 (m, 4H, CH₂α-chain2,3), 1.68 - 1.41 (m, 6H, CH₂β-chains1,2,3), 1.24 (m, 60H, 30xCH₂), 0.88 (t, ³J(H,H) = 6.7 Hz, 9H, 3xCH₃- chains1,2)
¹³C NMR (101 MHz, CDCl₃, 25 °C, TMS) δ 174.18, 173.02, 172.22, 129.58, 129.49, 113.74, 91.68, 73.17, 70.49, 68.84, 68.72, 55.22, 52.09, 36.73, 34.22, 34.10, 31.92, 29.70, 29.67, 29.66, 29.51, 29.36, 29.31, 29.27, 29.16, 25.58, 24.86, 22.69, 14.13, 14.12, 4.99, -1.60.

### 1-O-dibenzylphosphoryl-2-deoxy-6-O-(4-methoxybenzyl)- 4-O-tetradecanoyl 3-O-tetradecanoyl-2-tetradecanoylamino- α -D-glucopiranoside (compound 9, scheme 1 above).

Compound **8** (100 mg, 0.11 mmol) was dissolved in anhydrous CH₂Cl₂ (1.8 mL), then imidazolium triflate (83 mg, 0.32 mmol) and dibenzyl N,N-diisopropyl phosphoramidite (108 µl, 0.32 mmol) were added and the reaction was mixed at room temperature for 30 min. The solution was then cooled in ice bath, and mCPBA (93 mg, 0.54 mmol) was added. The reaction was mixed at room temperature overnight, then the mixture was diluted with CH₂Cl₂, washed with a saturated NaHCO₃ solution and saline. Organic layer was dried on anhydrous Na₂SO₄, filtered, and solvents were evaporated under vacuum. The raw product was purified by flash chromatography (petroleum ether-AE 8:2) yielding compound 9 as a brown solid (70 mg, 55%).
¹H-NMR: (400 MHz, CDCl₃, 25 °C, TMS): δ= 7.42 - 7.27 (m, 10H, H-benzyl) 7.17 (d, ³J(H,H) = 8.5 Hz, 2H, 2x H-ortho-PMB) 6.80 (d, ³J(H,H) = 8.4 Hz, 2H, 2x H-meta-PMB) 5.71 (dd, ³J(H,H) = 5.7, 3.3 Hz, 1H, H-1) 5.57 (d, ³J(H,H) = 9.1 Hz, 1H, NH) 5.24 - 5.14 (m, 2H, H-3, H-4) 5.11 - 4.95 (m, 4H, 2XCH₂ (benzyl)) 4.45 - 4.23 (m, 3H, CH₂ (PMB), H-2) 4.05 (m, 1H, H-5) 3.76 (s, 3H, OCH₃) 3.38 (d, ³J(H,H) = 3.4 Hz, 2H, H6a, H6b) 2.21 (t, ³J(H,H) = 7.6 Hz, 2H, CH₂α-chain1) 2.14 (t, ³J(H,H) = 7.5 Hz, 2H, CH₂α- chain2) 1.85 (m, 2H, CH₂α- chain3) 1.50 (m, 4H, CH₂β-chains1,2) 1.46 - 1.36 (m, 2H, CH₂β- chain3) 1.27 (m, 60H, 30xCH₂ chains) 0.88 (t, ³J(H,H) = 6.6 Hz, 9H, 3xCH₃ chains)
¹³C NMR (101 MHz, CDCl₃, 25 °C, TMS) δ 174.05 , 173.02, 171.89, 159.14, 129.48, 128.71, 128.64, 128.54, 128.04, 127.97, 127.93, 113.65, 90.78, 83.82, 75.02, 73.14, 70.99, 69.73, 69.21, 67.79, 55.17, 36.30, 34.14, 31.92, 31.10, 29.66, 29.51, 29.36, 29.16, 28.41, 25.36, 24.84, 22.71, 14.12, 5.16.

### 1-O-Phosphoryl -2-deoxy-4-O-tetradecanoyl 3-O-tetradecanoyl-2-tetradecanoylamino-α -D-glucopiranoside (FP11)

Compound **9** (25 mg, 0.021 mmol) was dissolved in anhydrous CH₂Cl₂ / MeOH 1:2 (1.3 mL), and added in catalytic amount Pd on activated coal. The reaction mixture was mixed at room temperature under H₂ atmosphere overnight. Triethylamine (80 µL) was added to the reaction mixture and the suspension was filtered with a syringe filter. The triethylammonium salt was dissolved in CH₂Cl₂ /MeOH1:2 (3 mL) and treated first with an Amberlite IRA 120 H⁺ exchange resin and then with an IR 120 Na⁺ exchange resin to remove triethylamine and form the sodium salt, yielding compound FP11 as a white solid (16 mg, 82%).
¹H-NMR: (400 MHz, CD₃OD, 25 °C, TMS) δ= 5.47 (dd, ³J(H,H) = 6.7, 3.2 Hz, 1H), 5.34 (t, ³J(H,H) = 10.0 Hz, 1H, H-3), 5.09 (t, ³J(H,H) = 9.9 Hz, 1H, H-4) 4.28 (dd, ³J(H,H) = 10.8, 2.5 Hz, 1H, H-2), 4.14 - 4.06 (m, 1H, H-5), 3.66 (m, 1H, H-6a) 3.56 (m, 1H, H-6b) 2.39 - 2.10 (m, 6H, 3x CH₂α-chains) 1.55 (s, 6H, 3xCH₂β-chains) 1.29 (m, 60H, 30xCH₂ catene) 0.89 (t, ³J(H,H) = 6.6 Hz, 9H, 3x CH₃-chains
¹³C NMR (101 MHz, CDCl₃, 25 °C, TMS) 131,65, 109,99, 92,21, 73,52, 34.14, 31.99, 31.96, 29.88, 29.80, 29.74, 29.71, 29.48, 29.43, 29.32, 24.94, 24.89, 22.71, 14.16, 14.13, 1.03, 1.02.
ESI-MS: [M]⁻ m/z = 888.6; found: m/z = 888.7

### 2-deoxy-4-O-tetradecanoyl 3-O-tetradecanoyl-2-tetradecanoylamino-β-D-glucopiranoside (compound 10, scheme 1 above).

Compound **8** (120 mg, 0.129 mmol) was dissolved in anhydrous CH₂Cl₂ /MeOH 1:2 (8 mL) and was added in catalytic amount Pd on activated coal. The reaction mixture was mixed at room temperature under H₂ atmosphere overnight. Solvents were evaporated under vacuum, yielding compound 10 as a white solid (100 mg, 95%).
¹H-NMR: (400 MHz, CDCl₃, 25 °C, TMS): δ 6.09 (d, ³J(H,H) = 9.1 Hz, 1H, H-1α) 5.34 (t, 3J(H,H) = 10.1 Hz, 1H, H-3) 5.02 (t, ³J(H,H) = 9.7 Hz, 1H, H-4) 4.21 (t, ³J(H,H) = 9.1 Hz, 1H, H-2) 4.04 (m, 1H, H-5) 3.67 (m, 1H, H-6a) 3.56 (m, 1H, H-6b) 2,25 (m, 4H, CH₂α-chains1,2) 2,11 (m, 4H, CH₂α-chain3) 1.52 (m, 6H, CH₂β-chains1,2,3) 1.23 (m, 60H, 30xCH₂) 0.86 (t, ³J(H,H) = 6.6 Hz, 9H, CH₃-chains1,2,3)
¹³C NMR (101 MHz, CDCl₃, 25 °C, TMS): δ 174.15, 173.64, 173.17, 91.38, 70.30, 69.67, 68.57, 68.47, 61.17, 52.46, 36.71, 34.20, 34.15, 31.92, 31.88, 29.73, 29.71, 29.68, 29.56, 29.55, 29.49, 29.41, 29.38, 29.37, 29.30, 29.20, 29.17, 25.61, 24.97, 24.93, 22.70, 22.68, 14.12, 14.11,

### 1,6-O-dibenzylphosphoryl-2-deoxy- 4-O-tetradecanoyl 3-O-tetradecanoyl-2-tetradecanoylamino- α -D-glucopiranoside (11).

Compound **10** (50 mg, 0.062 mmol) was dissolved in anhydrous CH₂Cl₂ (1.0 mL), then imidazolium triflate (72 mg, 0.28 mmol) and dibenzyl *N,N*-diisopropil fosforamidite (91 µl, 0.27 mmol) were added and the reaction was mixed at room temperature for 1.5 hours. The solution was then cooled in an ice bath and mCPBA (85 mg, 0.49 mmol). The reaction was mixed at room temperature overnight, then the mixture was diluted with CH₂Cl₂, washed with a saturated NaHCO₃ solution and saline. Organic layer was dried on anhydrous Na₂SO₄, filtered, and solvents were evaporated under vacuum. The raw product was purified by flash chromatography (petroleum ether-AE 8:2), yielding compound 11 as a brown compound (36 mg, 44%).
¹H-NMR: (400 MHz, CDCl₃, 25 °C, TMS): δ 7.44 - 7.21 (m, 20H, 4x Ar-H) 5.64 (dd, ³J(H,H) = 6.0, 3.3 Hz, 1H, H-1β) 5.61 (d, ³J(H,H) = 9.3 Hz, 1H, NH) 5.20 - 5.10 (m, 2H, H-3, H-5) 5.10 - 4.96 (m, 9H, 4x CH₂-Ph, H-4) 4.33 (t, ³J(H,H) = 9.8 Hz, 1H, H-2) 3.92 (m, 2H, H-6a, H-6b) 2,20 (m, 4H, CH₂α-chains1,2) 1.95 - 1.77 (m, 2H, CH₂α-chain3) 1.49 (m, 4H, CH₂β-chains1,2) 1.47 - 1.36 (m, 2H, CH₂β-chain3) 1.27 (m, 60H, 30xCH₂) 0.88 (t, ³J(H,H) = 6.6 Hz, 9H, CH₃-chains1,2,3)
¹³C NMR (101 MHz, CDCl₃, 25 °C, TMS) δ 173.95, 171.72, 151.63, 128.86, 128.77, 128.73, 128.54, 128.53, 128.49, 128.47, 128.10, 128.03, 127.96, 127.89, 109.99, 70.16, 69.88, 69.71, 69.44, 69.38, 66.67, 34.11, 31.92, 29.67, 29.51, 29.37, 29.31, 29.24, 29.16, 25.39, 24.85, 22.69, 14.13, 14.12.

### 1,6-O-Phosphoryl -2-deoxy-4-O-tetradecanoyl 3-O-tetradecanoyl-2-tetradecanoylamino- α -D-glucopiranoside (FP111)

Compound 11 (33 mg, 0.028 mmol) was dissolved in anhydrous CH₂Cl₂ / MeOH 1:2 (1.3 mL), and was added in catalytic amount Pd on activated coal. The reaction mixture was mixed at room temperature under H₂ atmosphere overnight. Then, triethylamine (80 µL) was added to the reaction mixture, and the suspension was filtered with a syringe filter. The triethylammonium salt was dissolved in CH₂Cl₂/MeOH 1:2 (3 mL) and treated first with an Amberlite IRA 120 H⁺ exchange resin and then with an IR 120 Na⁺ exchange resin to remove triethylamine and form the sodium salt, yielding FP111 as a white solid (20 mg, 68%).
¹H-NMR: (400 MHz, CD₃OD, 25 °C, TMS): 5.53 (dd, ³J(H,H) = 6.3, 3.2 Hz, 1H, H-1β) 5.32 (t, ³J(H,H) = 10.8 Hz, 1H, H-3) 5.14 (t, ³J(H,H) = 9.8 Hz, 1H, H-4) 4.32 (dt, ³J(H,H) = 10.9, 3.1 Hz, 1H, H-2) 4.28 - 4.21 (m, 1H, H-5) 4.12 - 3.97 (m, 2H, H-6a, H-6b) 2.48 - 2.06 (m, 6H, CH₂α-chains1,2,3) 1.71 - 1.47 (m, 6H, CH₂β-chains1,2,3) 1.29 (s, 60H, 30xCH₂) 0.90 (t, ³J(H,H) = 6.6 Hz, 9H, CH₃-chains1,2,3)
¹³C NMR (101 MHz, CD₃OD, 25 °C, TMS) δ 175.10, 172.94, 172.30, 127.68, 70.50, 69.44, 69.37, 68.31, 51.52, 48.20, 35.57, 33.65, 33.46, 31.71, 31.68, 29.47, 29.45, 29.28, 29.20, 29.12, 29.10, 29.02, 28.85, 28.80, 25.61, 24.50, 24.45, 22.35, 13.06, 13.05, -5.54. ESI-MS: [M+H]⁺ m/z = 968.6; found: m/z = 968.7

### Biology

### Preparation of stock solution of the compounds

In order to obtain a 10 mM stock solution of the concentrated compounds, a milligram of each compound was resuspended in the solvent indicated in the table below:

| **Compound** | **Molecular weight (g/mol)** | **Solvent** |
|---|---|---|
| FP11 | 934.18 | Ethanol: DMSO 1:1 |
| FP111 | 1058.12 | Ethanol: DMSO 1:1 |

The solution was stirred to complete dissolution of the compound.

### HEK-Blue^{™} cells assay

HEK-Blue ^{™} hTLR4 cells (InvivoGen) were cultured according to manufacturer's instruction.

Briefly, cells were cultured in DMEM high glucose medium supplemented with 10% fetal bovine serum (FBS), 2mM glutamine, antibiotics and 1XHEK-Blue ^{™} Selection (InvivoGen).

Cells were detached using a cell scraper, counted and seeded in a 96-well multiwell plate at a density of 4×10⁴ cells per well.

After overnight incubation (37 °C, 5% CO₂, 95% humidity), the culture medium was replaced by DMEM without Phenol Red, with addition of the compound to be tested. The cells were incubated overnight.

The SEAP-containing supernatants were collected and incubated with *para-*nitrophenylphosphate (pNPP) for 2-4 h in the dark at room temperature.

The wells' optical density was determined using a microplate reader set at 405 nm. The results were normalized with positive control (LPS alone) and expressed as a mean of percentage ± SEM of at least three independent experiments.

### MTT cell viability assay

HEK-Blue^{™} hTLR4 cells were grown in DMEM supplemented with 10% FBS, 2 mM glutamine and antibiotics.

Cells were seeded in 100 µL of DMEM without Phenol Red at a density of 4×10⁴ cells per well and incubated overnight (37 °C, 5% CO₂, 95% humidity).

Cells were treated with the higher dose of compound used in the previous experiments and incubated overnight. MTT solution (5 mg/mL in PBS) was added to each well, and after 3 h incubation, 0.1 N HCl in 2-propanol solution was used to dissolve formazan crystals.

Formazan concetration was determined by measuring the absorbance at 570 nm.

The results were normalized with untreated control (PBS) and expressed as the mean of percentage ± SEM of three independent experiments.

### Antibody-sandwich ELISA for the Detection of Binding of Compounds to MD-2

The method of antibody-sandwich ELISA for the detection of the binding of compounds to MD-2 was modified from a previous study [5].

A microtiter plate was coated overnight at 4°C with 100 µL/well of 5 µg/mL of chicken polyclonal anti-MD-2 antibodies, diluted in 50 mM Na₂CO₃ buffer, pH 9.6, and blocked with 1% BSA in PBS. After washing, 1 µM MD-2 with tested compounds was added and incubated for 2 h.

Mouse anti-MD-2 mAb (0.1 µg/mL 9B4) and goat antimouse IgG conjugated with HRP (0.1 µg/mL) in PBS were added, followed by detection at 420 nm after the addition of 100 µL of ABTS (Sigma).

Chicken anti-MD-2 polyclonal antibodies were prepared against recombinant MD-2 by GenTel (Madison, WI, USA), monoclonal mouse anti-MD-9B4 antibodies were from eBioscience (San Diego, CA, USA), and secondary goat antimouse IgG conjugated with horseradish peroxidase was from Santa Cruz Biotechnology (Santa Cruz, CA, USA).

### Fluorescence spectroscopy assay

Fluorescence was measured on PerkinElmer fluorimeter LS 55 (PerkinElmer, UK) as previously described ^{[5]}.

All measurements were done at 20°C in a 5 × 5 mm quartz glass cuvette (Hellma Suprasil, Müllheim, Germany). MD-2 protein (200 nM) and 1,1'-Bis(anilino)-4,4'-bis (naphtalene)-8,8' disulfonate (bis-ANS, 200 nM) were mixed and incubated until reaching stable relative fluorescence units (RFUs) emitted at 420-550 nm under excitation at 385 nm.

Compounds, at different concentrations, were then added, followed by relative fluorescence unit (RFU) measurement at 420-550 nm.

### LPS Displacement Assay

The ability of the compounds to displace LPS from MD-2 hydrophobic pocket was determined by ELISA.

A microtiter plate was coated overnight at 4°C with 100 µL/well of 5 µg/mL chicken polyclonal anti-MD-2 antibodies, diluted in 50 mM Na₂CO₃ buffer, pH 9.6, and blocked with 1% BSA in PBS. After washing, 1 µM MD-2 with biotin-labeled LPS was added and incubated for 2 h. After washing, the compounds were added at different concentrations and incubated for 1.5 h.

After washing, 0.5 µg/mL HRP-conjugated streptavidin (Sigma) in PBS was added, followed by detection at 420 nm after the addition of 100 µL of ABTS (Sigma). Chicken anti-MD-2 polyclonal antibodies were prepared against recombinant MD-2 by GenTel (Madison, WI, USA),

### Surface Plasmon Resonance (SPR) analysis

The binding affinity of the compounds to recombinant MD-2 was determined using a Biacore X100 with an NTA sensor chip (Biacore, GE Healthcare, Uppsala, Sweden). Briefly, 0.5 µM MD-2 (in 50 mM TRIS, 150 mM NaCl, 0.5% Tween 20, pH 7.5) was immobilized onto the sensor chip previously activated with 1 min pulse of 10 mM NiSO₄. The first flow cell was used as a reference surface to control nonspecific binding.

Both flow cells were incubated with the analyte (in PBS, 5% DMSO, 5% EtOH, pH 7.5) at a flow rate of 10 µL/min at 25 °C at increasing concentrations. The data were analyzed with Biacore Evaluation software. K_{D} values were calculated by global fitting of the equilibrium binding responses from various concentrations of analytes using a 1:1 Langmuir binding model.

## Claims

1. A compound of formula 1 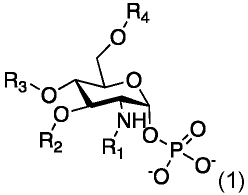
wherein R₁ is a saturated C₈-C₁₆ aliphatic chain having a =O on C₁, said chain being free from -OH substituents on C₃,
wherein R₂ is a saturated C₈-C₁₆ aliphatic chain having a =O on C₁, said chain being free from -OH substituents on C₃,
wherein R₃ is a saturated C₈-C₁₆ aliphatic chain having a =O on C₁, said chain being free from -OH substituents on C₃;
wherein R₄ is a hydrogen atom (H) or O-R₄ is a phosphate group (PO₄²⁻), and
wherein R1, R2 or R3 have no other substituents besides =O in C1 position.

2. The compound according to claim 1, having formula

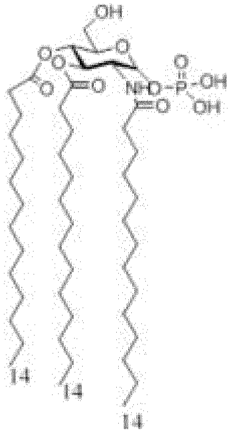

3. The compound according to any one of claims 1 to 2 for use in the treatment of diseases that require or benefit from an immunostimulation by activating the TLR4 receptor wherein said diseases are cancer, allergies or infective diseases.

4. The compound for use according to claim 3, wherein said compound is a vaccine adjuvant.

5. A vaccine composition comprising the compound according to any one of claims 1 to 2.

6. Vaccine composition according to claim 5, wherein said compound is the sole adjuvant present in said composition.

7. A pharmaceutical composition for use in the treatment of diseases that require or benefit from a TLR4 receptor activation comprising the compound according to any one of claims 1 to 2 and at least one pharmaceutically acceptable excipient, wherein said diseases are cancer, allergies or infective diseases.

8. A synthesis method for the preparation of compounds of formula 1 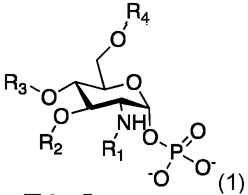 as defined in any one of claims 1 to 2 comprising the following steps:
1) transformation of the amine group of glucosamine into azide by reaction with trifluoromethansulfonic (triflic) azide;
2) selective protection of C₄ and C₆ hydroxyls on the sugar of said glucosamine forming *para-*methoxybenzylidene cyclic acetal by reaction with para-methoxybenzaldehyde dimethylacetal in the presence of camphorsulfonic acid (CSA) or by reaction of para-methoxybenzaldehyde in the presence of a catalyst acid;
3) protection of the anomeric carbon (C₁) as tert-butyldisilyl ether;
4) acylation of C₂ and C₃ positions with a C₈-C₁₆ linear chain carboxylic acid in the presence of condensing agents;
5) regioselective opening of the 4,6-paramethoxybenzylidene in reducing conditions to give *para-*methoxybenzyl ether in C₆ position via reaction with sodium cyanoborohydride and hydrochloric acid;
6) acylation of the C₄ position by reaction with a C₈-C₁₆ linear chain carboxylic acid in the presence of condensing agents;
7) hydroxyl deprotection on C₁;
8) phosphorylation of position C₁ by reaction with dibenzyl phosphoramidite and imidazolium triflate, followed by phosphorous oxidation by the use of meta-chloroperbenzoic acid; and
9) deblocking the para-methoxybenzyl ether in C₆ and simultaneously deprotecting the benzyl groups on the phosphates by catalytic hydrogenation in the presence of a palladium-carbon catalyst, or
10) deblocking the *para*-methoxybenzyl ether in C₆ by catalytic hydrogenation with a Pd/C catalyst;
11) phosphorylation in C₆ by reaction with dibenzyl-*N,N*-diisopropylphosphoramidite and imidazolium triflate, followed by phosphorous oxidation by the use of *meta*-chloroperbenzoic acid; and
12) deprotection of benzyl groups on the phosphates by catalytic hydrogenation with a Pd/C catalyst.

## Patentansprüche

1. Verbindung der Formel 1 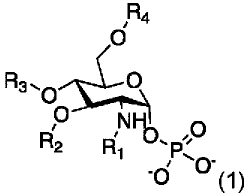
wobei R₁ eine gesättigte aliphatische C₈-C₁₆-Kette ist, die ein =O an C₁ aufweist, wobei die Kette frei von -OH-Substituenten an C₃ ist,
wobei R₂ eine gesättigte aliphatische C₈-C₁₆-Kette ist, die ein =O an C₁ aufweist, wobei die Kette frei von -OH-Substituenten an C₃ ist,
wobei R₃ eine gesättigte aliphatische C₈-C₁₆-Kette ist, die ein =O an C₁ aufweist, wobei die Kette frei von -OH-Substituenten an C₃ ist;
wobei R₄ ein Wasserstoffatom (H) ist oder O-R₄ eine Phosphatgruppe (PO₄²-) ist und
wobei R1, R2 oder R3 außer =O in der C1-Position keine anderen Substituenten aufweisen.

2. Verbindung nach Anspruch 1, aufweisend die Formel

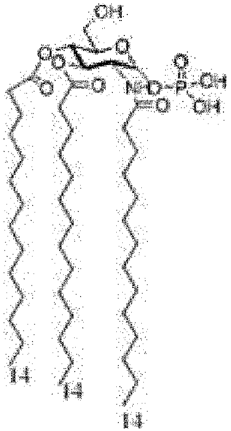

3. Verbindung nach einem der Ansprüche 1 bis 2 zur Verwendung bei der Behandlung von Krankheiten, die eine Immunstimulation durch Aktivierung des TLR4-Rezeptors erfordern oder von dieser profitieren, wobei es sich bei den Krankheiten um Krebs, Allergien oder Infektionskrankheiten handelt.

4. Verbindung zur Verwendung nach Anspruch 3, wobei die Verbindung ein Impfstoffadjuvans ist.

5. Impfstoffzusammensetzung, umfassend die Verbindung nach einem der Ansprüche 1 bis 2.

6. Impfstoffzusammensetzung nach Anspruch 5, wobei die Verbindung das einzige in der Zusammensetzung vorhandene Adjuvans ist.

7. Pharmazeutische Zusammensetzung zur Verwendung bei der Behandlung von Krankheiten, die die Aktivierung eines TLR4-Rezeptors erfordern oder von dieser profitieren, umfassend die Verbindung nach einem der Ansprüche 1 bis 2 und mindestens einen pharmazeutisch verträglichen Hilfsstoff, wobei es sich bei den Krankheiten um Krebs, Allergien oder Infektionskrankheiten handelt.

8. Syntheseverfahren für die Herstellung von Verbindungen der Formel 1 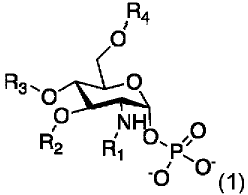 wie nach einem der Ansprüche 1 bis 2 definiert, umfassend die folgenden Schritte:
1) Umwandeln der Amingruppe von Glucosamin in Azid durch Reaktion mit Trifluormethansulfonsäure (Trifllinsäure);
2) selektives Protektieren von C₄- und C₆-Hydroxylen an dem Zucker des Glucosamin ausbildenden cyclischen *Para*-Methoxybenzylidenacetal durch Reaktion mit *Para*-Methoxybenzaldehyddimethylacetal in Gegenwart von Camphersulfonsäure (CSA) oder durch Reaktion von Para-Methoxybenzaldehyd in Gegenwart einer Katalysatorsäure;
3) Protektieren des anomeren Kohlenstoffs (C₁) als tert-Butyldisilylether;
4) Acylieren von C₂- und C₃-Positionen mit einer geradkettigen C₈-C₁₆-Carbonsäure in Gegenwart von Kondensationsmitteln;
5) regioselektives Öffnen des 4,6-Paramethoxybenzylidens bei reduzierenden Bedingungen, um Para-Methoxybenzylether in der C₆-Position zu ergeben, über Reaktion mit Natriumcyanoborhydrid und Salzsäure;
6) Acylieren der C₄-Position durch Reaktion mit einer geradkettigen C₈-C₁₆-Carbonsäure in Gegenwart von Kondensationsmitteln;
7) Hydroxyldeprotektieren an C₁;
8) Phosphorylieren der C₁-Position durch Reaktion mit Dibenzylphosphoramidit und Imidazoliumtriflat, gefolgt von Phosphoroxidation durch die Verwendung von *Meta*-Chlorperbenzoesäure; und
9) Deblockieren des *Para*-Methoxybenzylethers in C₆ und gleichzeitiges Deprotektieren der Benzylgruppen an den Phosphaten durch katalytische Hydrierung in Gegenwart eines Palladiumkohlenstoffkatalysators oder
10) Deblockieren des *Para*-Methoxybenzylethers in C₆ durch katalytische Hydrierung mit einem Pd/C-Katalysator;
11) Phosphorylieren in C₆ durch Reaktion mit Dibenzyl-*N,N-*Diisopropylphosphoramidit und Imidazoliumtriflat, gefolgt von Phosphoroxidation durch die Verwendung von Meta-Chlorperbenzoesäure; und
12) Deprotektieren der Benzylgruppen an den Phosphaten durch katalytische Hydrierung mit einem Pd/C-Katalysator.

## Revendications

1. Composé de formule 1 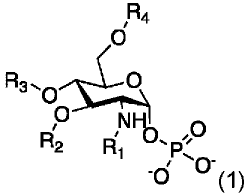
où R₁ est une chaîne aliphatique saturée en C₈-C₁₆ ayant un =O sur C₁, ladite chaîne étant exempte de substituants -OH sur C₃,
où R₂ est une chaîne aliphatique saturée en C₈-C₁₆ ayant un =O sur C₁, ladite chaîne étant exempte de substituants -OH sur C₃,
où R₃ est une chaîne aliphatique saturée en C₈-C₁₆ ayant un =O sur C₁, ladite chaîne étant exempte de substituants -OH sur C₃ ;
où R₄ est un atome d'hydrogène (H) ou O-R₄ est un groupe phosphate (PO₄²-), et
où R1, R2 ou R3 n'ont pas d'autres substituants que =O en position C1.

2. Composé selon la revendication 1, ayant la formule

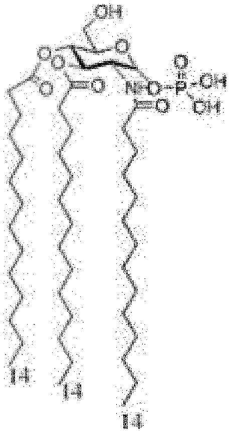

3. Composé selon l'une quelconque des revendications 1 à 2, destiné à être utilisé dans le traitement de maladies qui nécessitent une immunostimulation par activation du récepteur TLR4, ou en bénéficient, où lesdites maladies sont un cancer, des allergies ou des maladies infectieuses.

4. Composé destiné à être utilisé selon la revendication 3, où ledit composé est un adjuvant de vaccin.

5. Composition de vaccin comprenant le composé selon l'une quelconque des revendications 1 à 2.

6. Composition de vaccin selon la revendication 5, où ledit composé est le seul adjuvant présent dans ladite composition.

7. Composition pharmaceutique destinée à être utilisée dans le traitement de maladies qui nécessitent une activation du récepteur TLR4, ou en bénéficient, comprenant le composé selon l'une quelconque des revendications 1 à 2 et au moins un excipient pharmaceutiquement acceptable, où lesdites maladies sont un cancer, des allergies ou des maladies infectieuses.

8. Procédé de synthèse pour la préparation de composés de formule 1 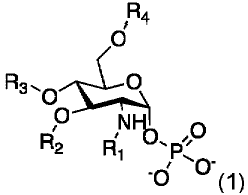 tels que définis dans l'une quelconque des revendications 1 à 2, comprenant les étapes suivantes :
1) transformation du groupe amine de glucosamine en azoture par réaction avec de l'azoture trifluorométhanesulfonique (triflique) ;
2) protection sélective des hydroxyles en C₄ et C₆ sur le sucre de ladite glucosamine formant l'acétal cyclique de *para*-méthoxybenzylidène par réaction avec le diméthylacétal de para-méthoxybenzaldéhyde en présence d'acide camphorsulfonique (CSA) ou par réaction de para-méthoxybenzaldéhyde en présence d'un acide catalyseur ;
3) protection du carbone anomérique (C₁) sous forme d'éther de tert-butyldisilyle ;
4) acylation des positions C₂ et C₃ avec un acide carboxylique à chaîne linéaire en C₈-C₁₆ en présence d'agents de condensation ;
5) ouverture régiosélective du 4,6-paraméthoxybenzylidène dans des conditions réductrices pour donner de l'éther para-méthoxybenzylique en position C₆ par réaction avec du cyanoborohydrure de sodium et de l'acide chlorhydrique ;
6) acylation de la position C₄ par réaction avec un acide carboxylique à chaîne linéaire en C₈-C₁₆ en présence d'agents de condensation ;
7) déprotection de l'hydroxyle sur C₁ ;
8) phosphorylation de la position C₁ par réaction avec du dibenzylphosphoramidite et du triflate d'imidazolium, en faisant suivre par une oxydation du phosphore par l'utilisation d'acide méta-chloroperbenzoïque ; et
9) déblocage de l'éther *para*-méthoxybenzylique en C₆ et déprotection simultanée des groupes benzyle sur les phosphates par hydrogénation catalytique en présence d'un catalyseur palladium-carbone, ou
10) déblocage de l'éther *para*-méthoxybenzylique en C₆ par hydrogénation catalytique avec un catalyseur Pd/C ;
11) phosphorylation en C₆ par réaction avec du dibenzyl-*N,N-*diisopropylphosphoramidite et du triflate d'imidazolium, en faisant suivre par une oxydation du phosphore par l'utilisation d'acide *méta*-chloroperbenzoïque ; et
12) déprotection des groupes benzyle sur les phosphates par hydrogénation catalytique avec un catalyseur Pd/C.
